# EUROPEAN PATENT SPECIFICATION

(11) **EP 3 622 951 B1**
(45) Date of publication and mention of the grant of the patent: **11.10.2023**
(21) Application number: 18797925.7
(22) Date of filing: 11.05.2018
(51) Int. Cl.: A61K 31/215, A23D 9/00, A23L 33/115, A61P 25/28, A23L 33/12

(54) **AGENT FOR RAISING TOTAL KETONE CONCENTRATION, OIL AND FAT COMPOSITION, PHARMACEUTICAL COMPOSITION, AND FOOD PRODUCT COMPOSITION**
MITTEL ZUR ERHÖHUNG DER GESAMTKETONKONZENTRATION, ÖL- UND FETTZUSAMMENSETZUNG, PHARMAZEUTISCHE ZUSAMMENSETZUNG UND NAHRUNGSMITTELZUSAMMENSETZUNG
AGENT POUR AUGMENTER LA CONCENTRATION TOTALE DE CÉTONE, COMPOSITION D'HUILE ET DE GRAISSE, COMPOSITION PHARMACEUTIQUE ET COMPOSITION DE PRODUIT ALIMENTAIRE

(30) Priority: 12.05.2017 JP 2017095475
(43) Date of publication of application: 18.03.2020
(73) Proprietor: The Nisshin OilliO Group, Ltd., Tokyo 104-8285 (JP)
(72) Inventor: TAKAGI Tetsuo, Yokohama-shi Kanagawa 235-8558 (JP); HONDA Kazumitsu, Yokohama-shi Kanagawa 235-8558 (JP)
(74) Representative: KATZAROV S.A.
(86) International application number: PCT/JP2018/018385
(87) International publication number: WO 2018/207921

(56) References cited:
- WO-A1-2011/078367
- WO-A1-2016/010102
- JP-A- H08 208 467
- US-A1- 2012 328 760
- NAFAR, F. et al.: "Coconut oil protects cortical neurons from amyloid beta toxicity by enhancing signaling of cell survival pathways", Neurochemistry International, vol. 105, 2017, pages 64-79, XP055562993, ISSN: 0197-0186
- IWASA, YOSHIE et al.: "Review on Medium Chain Triglyceride (MCT) Emulsion", Japanese journal of parenteral and enteral nutrition, vol. 12, no. 9, 1990, pages 1115-1120, XP009517897, ISSN: 0388-127X
- MCCARTY, M. F. et al.: "Laurie acid-rich medium-chain triglycerides can substitute for other oils in cooking applications and may have limited pathogenicity", Open Heart, vol. 3, no. 2, 2016, pages 1-5, XP055563035, ISSN: 2053-3624
- NONAKA, Y. et al.: "Lauric Acid Stimulates Ketone Body Production in the KT-5 Astrocyte Cell Line", Journal of Oleo Science, vol. 65, no. 8, 2016, pages 693-699, XP055654817, ISSN: 1345-8957
- NONAKA, YUDAI: "Medium-chain fatty acids stimulate ketone body production in the KT-5 astrocyte cell line", 55st Congress of Japanese Society of Physical Therapy, September 2016 (2016-09), page 199, XP009517950,
- BOROS, L. A. D. et al.: "Binary mixtures of fatty acid ethyl esters: Solid- liquid equilibrium", Fluid Phase Equilibria, vol. 427, 2016, pages 1-8, XP029713115, ISSN: 0378-3812
- OLIVEIRA DE SOUZA, L. I. et al.: "The chemical composition and trypanocidal activity of volatile oils from Brazilian Caatinga plants", Biomedicine & Pharmacotherapy, vol. 96, December 2017 (2017-12), pages 1055-1064, XP055563157, ISSN: 0753-3322
- BAUER, L. C. et al.: "Chemical characterization of pressed and refined licuri (Syagrus coronata) oils", Acta Scientiarum. Technology, vol. 35, no. 4, 2013, pages 771-776, XP055563160, ISSN: 1806-2563

## Description

### TECHNICAL FIELD

The present invention relates to a pharmaceutical composition, and a food product composition for use in treatment or prevention of dementia or age-associated cognitive decline.

### BACKGROUND ART

In recent years, the number of patients suffering from dementia which may be caused by reduced neuronal metabolism (for example, Alzheimer's dementia) or having the onset of cognitive decline which may be caused by reduced neuronal metabolism in the course of aging (for example, age-associated memory impairment (AAMI)) is increasing. This represents a serious social problem. Accordingly, various methods of preventing or treating symptoms of these disorders have been developed.

As methods of preventing or treating dementia which may be caused by reduced neuronal metabolism or age-associated cognitive decline, a system using metabolism of medium-chain fatty acids into ketone bodies in the liver and a system using metabolism of medium-chain fatty acids into ketone bodies in the brain have been contemplated.

For a system using metabolism in the liver, ingestion of a medium-chain fatty acid having a carbon number of 6 and the like is shown to be a potentially effective method of preventing or treating Alzheimer's dementia which may be caused by reduced neuronal metabolism (for example, Patent Document 1). Further, ingestion of a medium-chain fatty acid having a carbon number of 8 and the like is shown to be a potentially effective method of preventing or treating age-associated memory impairment (AAMI) (for example, Patent Document 2).

Further, for a system using metabolism in the brain, lauric acid (a medium-chain fatty acid having a carbon number of 12) among medium-chain fatty acids is shown to promote ketone bodies production in the brain, and to thus be likely effective for prevention or improvement of Alzheimer's dementia (Nonpatent document 1).

In either system, Alzheimer's dementia and age-associated memory impairment can be prevented or ameliorated assumedly via a mechanism in which ketone bodies produced from medium-chain fatty acids are converted into energy within the brain, so as to supply energy to the brain suffering an abnormality in glucose metabolism.

Patent application 2012/328760 A1 discloses an emulsion comprising an oil phase having particular ratios of capric acid, lauric acid and myristic acid to caprylic acid. The weight ratio of capric acid. The weight ratios are as follows: capric acid is 20 to 97, lauric acid 28 to 6000, myristic acid 11 to 2100 and caprylic acid 100. Preferably, the weight ratios are as follows: capric acid 20 to 95, lauric acid 28 to 600, myristic acid 11 to 1000 and caprylic acid 100. Even more preferably the weight ratios are as follows: capric acid 31 to 95, lauric acid 30 to 250, myristic acid 11 to 80 and caprylic acid 100. The emulsion is disclosed as being stable and suitable for addition of an oil-soluble component such as a capsinoid. This document is silent with respect to treatment and prevention of dementia or age-associated cognitive decline.

Nafar et al., Neurochemistry International 105 (2017) 64-79 discloses the usefulness of coconut oil, octanoic acid, lauric acid or a combination of octanoic and lauric acid for use in the treatment of Alzheimer's disease. D2 further describes a composition comprising octanoic and lauric acid in a proportion of octanoic acid _ lauric acid of 0.02 mM : 0.05 mM, i.e. approximately 22 and 78% by mass, respectively. This document does not disclose the present concentrations of C8, C10 and C12 fatty acids.

McCarty et al., Open Heart 2016:3, 1-3 teaches to combine C8 and C10 medium chain fatty acids with lauric aid. Enriching MCT with lauric acid is suggested to produce a more sustained and moderate rise in plasma ketone bodies, as opposed to the large episodic rise and falls which ingestion of standard MCTs would tend to produce. This document does not disclose the present concentrations of C8, C10 and C12 fatty acids.

Also cited are the following documents:
Patent Document 1: Japanese Patent No. 5701245
Patent Document 2: Japanese Patent No. 5847693
Non-Patent Document 1: J. Oleo Sci. 65,(8) 693-699(2016)

### DISCLOSURE OF THE INVENTION

### Problems to be Solved by the Invention

In the conventional technologies, an increase in the amount of ketone body production (the concentration of total ketone bodies) due to ingestion of a medium-chain fatty acid can, however, be maintained for only about 1 to 2 hours after the ingestion of the medium-chain fatty acid. Therefore, a technology has been desired which can maintain the concentration of total ketone bodies in the brain over a longer period of time.

The present invention is made in order to solve the above problem. An object of the present invention is to provide a technology capable of treatment or prevention of dementia or age-associated cognitive decline by maintaining the concentration of total ketone bodies in the brain for a prolonged period of time.

### Means for Solving the Problems

Surprisingly, the present investors found that ingestion of medium-chain fatty acids having particular carbon numbers at a particular ratio can maintain the concentration of total ketone bodies in the brain for a prolonged period of time. Thus, the present invention has been completed. Specifically, the present invention can provide the following.
(1) A pharmaceutical composition for use in treatment or prevention of dementia or age-associated cognitive decline, the pharmaceutical composition comprising
   a) an agent for use in a method of increasing the concentration of total ketone bodies, the agent comprising 40 mass% or more to 75 mass% or less of a medium-chain fatty acid having a carbon atom number of 8, 0 mass% or more to less than 25 mass% of a medium-chain fatty acid having a carbon number of 10, and 25 mass% or more to 60 mass% or less of a medium-chain fatty acid having a carbon number of 12 with respect to the total amount of medium-chain fatty acids; and/or
   b) an oil and/or fat composition comprising an oil and/or fat having constituent fatty acids including 50 mass% or more to 75 mass% or less of a medium-chain fatty acid having a carbon number of 8, 0 mass% or more to less than 25 mass% of a medium-chain fatty acid having a carbon number of 10, and 25 mass% or more to 50 mass% or less of a medium-chain fatty acid having a carbon number of 12 with respect to the total amount of medium-chain fatty acids, wherein the medium-chain fatty acids are present in the form of acylglycerols.
(8) A food product composition for use in treatment or prevention of dementia or age-associated cognitive decline, the food product composition comprising
   a) an agent for use in a method of increasing the concentration of total ketone bodies, the agent comprising 40 mass% or more to 75 mass% or less of a medium-chain fatty acid having a carbon atom number of 8, 0 mass% or more to less than 25 mass% of a medium-chain fatty acid having a carbon number of 10, and 25 mass% or more to 60 mass% or less of a medium-chain fatty acid having a carbon number of 12 with respect to the total amount of medium-chain fatty acids; and/or
   b) oil and/or fat composition comprising an oil and/or fat having constituent fatty acids including 50 mass% or more to 75 mass% or less of a medium-chain fatty acid having a carbon number of 8, 0 mass% or more to less than 25 mass% of a medium-chain fatty acid having a carbon number of 10, and 25 mass% or more to 50 mass% or less of a medium-chain fatty acid having a carbon number of 12 with respect to the total amount of medium-chain fatty acids, wherein the medium-chain fatty acids are present in the form of acylglycerols.

### Effects of the Invention

The present invention can provide a technology capable of treatment or prevention of dementia or age-associated cognitive decline by maintaining the concentration of total ketone bodies in the brain for a prolonged period of time.

### BRIEF DESCRIPTION OF THE DRAWINGS

Fig. 1 shows the effects of the agent for increasing the concentration of total ketone bodies on the concentration of total ketone bodies in the hippocampus.
Fig. 2 shows the effects of the agent for increasing the concentration of total ketone bodies on the concentration of total ketone bodies in the hippocampus.

### PREFERRED MODE FOR CARRYING OUT THE INVENTION

### <Agent for increasing concentration of total ketone bodies>

The agent for increasing the concentration of total ketone bodies includes a medium-chain fatty acid. Hereinafter, the term "medium-chain fatty acid" may also be referred to as an "MCFA."

### (Medium-chain fatty acid (MCFA))

An MCFA is a linear saturated fatty acid having a carbon number of 6 to 12, and is an oil and/or fat component contained in common food products and the like (for example, edible oil and/or fat products, dairy products, or the like). The agent for increasing the concentration of total ketone bodies according to the embodiment of the present invention includes 30 mass% or more to 75 mass% or less of an MCFA having a carbon number of 8, 0 mass% or more to less than 25 mass% of an MCFA having a carbon number of 10, and 25 mass% or more to 60 mass% or less of an MCFA having a carbon number of 12 with respect to the total amount of MCFAs contained in the agent. Hereinafter, the MCFA having a carbon number of 8, the MCFA having a carbon number of 10, and the MCFA having a carbon number of 12 may be collectively referred to as the "3 types of MCFAs for use in the present invention."

After intensive studies, the present inventors found that ingestion of the 3 types of MCFAs for use in the present invention at the aforementioned percentages can maintain the concentration of total ketone bodies in the brain for a prolonged period of time (for example, for 4 hours or longer). In contrast, ingestion of a preparation which did not meet the aforementioned percentages (for example, ingestion of only an MCFA having a carbon number of 8) increased the concentration of total ketone bodies only temporarily, and was able to maintain the effect thereof only for 1 to 2 hours after the ingestion. One possible reason why such a result was obtained may be as follows. Specifically, a smaller proportion of an MCFA having a carbon number of 12 will be metabolized into ketone bodies in the liver as compared with an MCFA having a carbon number of 8 and an MCFA having a carbon number of 10 as other medium-chain fatty acids. Therefore, an MCFA having a carbon number of 12 which is not metabolized into ketone bodies will flow into the blood stream. Such an MCFA having a carbon number of 12 may be metabolized into ketone bodies in astrocytes when it reaches the brain (see J. Oleo Sci. 65(8): 693-699 (2016)). Thus, it is thought that a combination of metabolic pathways and MCFAs having different metabolic rates can maintain the concentration of total ketone bodies in the brain for a prolonged period of time (for example, for 4 hours or longer).

According to the present invention, the concentration of total ketone bodies in the brain tends not to decrease even if an energy source is not ingested between meals. This can be suitably used to prevent or treat Alzheimer's dementia or age-associated cognitive decline. It is noted that the term "between meals" as used in the present invention means a time between a meal and the next meal, and is usually for 6 to 7 hours. Any activities other than having a meal may be allowed between meals, and the person in question may be awake or asleep.

In view of more easily maintaining the concentration of total ketone bodies for a prolonged period of time, the agent for increasing the concentration of total ketone bodies includes 40 mass% or more, more preferably 45 mass% or more, and even more preferably 55 mass% or more of an MCFA having a carbon number of 8 with respect to the total amount of MCFAs contained in the agent. In view of sufficiently blending MCFAs other than an MCFA having a carbon number of 8, the agent for increasing the concentration of total ketone bodies includes 75 mass% or less, more preferably 69 mass% or less, even more preferably 67 mass% or less, and yet more preferably 66 mass% or less of an MCFA having a carbon number of 8 with respect to the total amount of MCFAs contained in the agent.

In view of more easily maintaining the concentration of total ketone bodies for a prolonged period of time, the agent for increasing the concentration of total ketone bodies includes 0 mass% or more of an MCFA having a carbon number of 10 with respect to the total amount of MCFAs contained in the agent. The agent for increasing the concentration of total ketone bodies does not need to include an MCFA having a carbon number of 10. In view of sufficiently blending MCFAs other than an MCFA having a carbon number of 10, the agent for increasing the concentration of total ketone bodies includes less than 25 mass%, more preferably 20 mass% or less, even more preferably 15 mass% or less, and yet more preferably 10 mass% or less of an MCFA having a carbon number of 10 with respect to the total amount of MCFAs contained in the agent. In view of more readily achieving the effects of the present invention, the agent for increasing the concentration of total ketone bodies does not need to include an MCFA having a carbon number of 10.

In view of more easily maintaining the concentration of total ketone bodies for a prolonged period of time, the agent for increasing the concentration of total ketone bodies includes 25 mass% or more, preferably 31 mass% or more, more preferably 33 mass% or more, and even more preferably 34 mass% or more of an MCFA having a carbon number of 12 with respect to the total amount of MCFAs contained in the agent. In view of sufficiently blending MCFAs other than an MCFA having a carbon number of 12, the agent for increasing the concentration of total ketone bodies includes 60 mass% or less, more preferably 55 mass% or less, and even more preferably 50 mass% or less of an MCFA having a carbon number of 12 with respect to the total amount of MCFAs contained in the agent.

In view of achieving particularly easy maintenance of the concentration of total ketone bodies for a prolonged period of time, the agent for increasing the concentration of total ketone bodies includes 60 mass% or more to 70 mass% or less of an MCFA having a carbon number of 8 and 30 mass% or more to 40 mass% or less of an MCFA having a carbon number of 12 with respect to the total amount of MCFAs contained in the agent. More preferably, the agent for increasing the concentration of total ketone bodies consists of 60 mass% or more to 70 mass% or less of an MCFA having a carbon number of 8 and 30 mass% or more to 40 mass% or less of an MCFA having a carbon number of 12 with respect to the total amount of MCFAs contained in the agent.

Examples of an MCFA having a carbon number of 8 include caprylic acid (n-octanoic acid). Examples of an MCFA having a carbon number of 10 include capric acid (n-decanoic acid). Examples of an MCFA having a carbon number of 12 include lauric acid.

There is no particular limitation for the forms of MCFAs contained in an agent for increasing the concentration of total ketone bodies. They may be medium-chain fatty acids themselves, or may be of fatty acid precursors (for example, salts, esters (acylglycerols and the like described below)) which may be converted into medium-chain fatty acids in the living body, or may be a mixture thereof.

MCFAs may be obtained by, for example, hydrolyzing palm kernel oil or coconut oil followed by performing refinement. Commercially available products or reagents may also be used as MCFAs.

An MCFA is usually ingested into the body in the form of a fatty acid precursor, more specifically in the form of an acylglycerol in which an MCFA is bound with glycerin via an ester bond. An ingested acylglycerol is known to be decomposed and absorbed in the digestive tract to release an MCFA, which is then converted into energy in the liver or the brain. In view of higher safety and the like, MCFAs contained in the agent for increasing the concentration of total ketone bodies are preferably in the form of acylglycerols.

An acylglycerol, which has a structure in which a fatty acid is bound with glycerin via an ester bond, is present as any of 3 types of forms (monoacylglycerol, diacylglycerol, and triacylglycerol) which differ in the number of fatty acids bound with glycerin. The acylglycerol for use in the present invention may be any of the above 3 types of forms. There is no particular limitation for the type and amount of the acylglycerol contained in an agent for increasing the concentration of total ketone bodies as long as the percentages of the 3 types of MCFAs with respect to the total amount of MCFAs for use in the present invention contained in the agent for increasing the concentration of total ketone bodies satisfy the aforementioned requirements.

The acylglycerol for use in the present invention is preferably triacylglycerol because it is close to a form used in common food products. Further, in the present invention, the fatty acids of diacylglycerol and triacylglycerol may be of the same type, or may be of different types. In a case of an acylglycerol composed of different types of fatty acids, there is no particular limitation for the position at which each fatty acid is bound to glycerin. Further, a fatty acid other than the 3 types of MCFAs for use in the present invention (for example, an MCFA other than the 3 types of MCFAs for use in the present invention such as caproic acid (n-hexanoic acid), pentanoic acid, heptanoic acid, and nonanoic acid; a short-chain fatty acid having a carbon number of 4 (butanoic acid); a long-chain fatty acid having a carbon number of 14 to 22; and the like) may be contained as a constituent fatty acid of an acylglycerol.

An acylglycerol which does not include the 3 types of MCFAs for use in the present invention as constituent fatty acids may further be blended in the agent for increasing the concentration of total ketone bodies. In that case, the percentages of the 3 types of MCFAs for use in the present invention with respect to the total amount of MCFAs among all the constituent fatty acids of the acylglycerol blended in the agent for increasing the concentration of total ketone bodies should fall within the aforementioned ranges.

There is no particular limitation for a method of manufacturing acylglycerol, but it may be obtained by, for example, performing an esterification reaction of an MCFA derived from palm kernel oil or coconut oil with glycerin. Methods for performing an esterification reaction may include, for example, a method involving a reaction under pressure without a catalyst nor a solvent, a method involving a reaction with a synthesis catalyst such as sodium methoxide, a method involving a reaction using lipase as a catalyst, and the like.

In view of easily obtaining a safer agent for increasing the concentration of total ketone bodies, a triglyceride including any one or more of the 3 types of MCFAs for use in the present invention and a long-chain fatty acid (for example, a linear long-chain fatty acid having a carbon number of 14 to 22) as constituent fatty acids thereof, i.e., a medium- to long-chain fatty-acid triglyceride; or a triglyceride in which constituent fatty acids thereof are all MCFAs, i.e., a medium-chain fatty-acid triglyceride, is preferred as an acylglycerol for use in the present invention. As an acylglycerol for use in the present invention, particularly preferred is a medium-chain fatty-acid triglyceride in which constituent fatty acids thereof are each any one of the 3 types of MCFAs for use in the present invention. Hereinafter, the term "medium- to long-chain fatty acid triglyceride" may be referred to as "MLCT", and the term "medium-chain fatty-acid triglyceride" may also be referred to as "MCT."

A type(s) of an MLCT and/or an MCT is/are preferably included as an MCFA contained in the agent for increasing the concentration of total ketone bodies. Further, 30 mass% or more of an MCT is preferably included with respect to the total of an MLCT and the MCT. More preferably, 60 mass% or more of an MCT is included, and even more preferably 80 mass% or more of an MCT is included, and most preferably only an MCT is included with respect to the total of an MLCT and the MCT. In particular, the agent for increasing the concentration of total ketone bodies preferably consists of an MCT only, and most preferably consists of an MCT including only the 3 types of MCFAs in the present invention.

The agent for increasing the concentration of total ketone bodies may consist of MCFAs (MCFAs themselves, a fatty acid precursor (an MLCT, an MCT, and the like), or a mixture thereof), or may include another component other than an MCFA along with the MCFA. The agent for increasing the concentration of total ketone bodies preferably consists of MCFAs, and more preferably consists of the 3 types of MCFAs for use in the present invention.

When a component other than the 3 types of MCFAs for use in the present invention is included in the agent for increasing the concentration of total ketone, the lower limit of the blending amount of the 3 types of MCFAs for use in the present invention (MCFAs themselves, a fatty acid precursor of MCFAs (an MLCT, an MCT, and the like), or a mixture thereof) contained in the agent for increasing the concentration of total ketone bodies is preferably 33 mass% or more, more preferably 50 mass% or more with respect to the agent for increasing the concentration of total ketone bodies. The upper limit is preferably 99 mass% or less, more preferably 90 mass% or less with respect to the agent for increasing the concentration of total ketone bodies. Within the above ranges, the 3 types of MCFAs for use in the present invention can effectively increase the concentration of total ketone bodies.

There is no particular limitation for a component other than the 3 types of MCFAs for use in the present invention, which can be included in the agent for increasing the concentration of total ketone bodies as long as it does not impair the effects of these MCFAs. Examples of such a component include proteins, lipids, available carbohydrates, vitamins, minerals, sweetening agents, anti-oxidative agents, emulsifying agents, various seasonings, swelling agents, flavoring agents, coloring agents, and the like. The types and blending amounts of these components can be appropriately selected depending on the desired effect.

The contents of the 3 types of MCFAs for use in the present invention contained in the agent for increasing the concentration of total ketone bodies can be determined by gas chromatography.

### (Action of increasing concentration of total ketone bodies)

The agent for increasing the concentration of total ketone bodies can increase the concentration of total ketone bodies in the brain.

In general, the term "total ketone body" is a generic name for acetone, acetoacetic acid, and 3-hydroxybutyric acid. However, acetone is not used as an energy source, and is discharged through expiration. Therefore, the term "total ketone body" as used in the present invention means a generic name for acetoacetic acid and 3-hydroxybutyric acid, and thus the "concentration of total ketone bodies" means the total concentration of these two types of compounds. The concentration of total ketone bodies can be determined with a commercially available kit based on the enzyme cycling method using 3-hydroxybutyric acid dehydrogenase.

The phrase "increasing the concentration of total ketone bodies" as used in the present invention means that the concentration of total ketone bodies in the brain is increased by ingestion of the agent for increasing the concentration of total ketone bodies according to an embodiment of the present invention, as compared to a level before the ingestion. According to an embodiment of the invention, ingestion of an agent for increasing the concentration of total ketone bodies not only can increase the concentration of total ketone bodies but also can maintain that increased concentration for a prolonged period of time. According to an embodiment of the present invention, for example, the concentration of total ketone bodies can be maintained for 4 hours or longer (preferably 6 hours or longer, more preferably 8 hours or longer) after ingestion of an agent for increasing the concentration of total ketone bodies. The phrase "maintaining the concentration of total ketone bodies" as used in the present invention means that the concentration of total ketone bodes increased after ingestion of an agent for increasing the concentration of total ketone bodies does not easily decrease over time. In an embodiment of the present invention, the concentration of total ketone bodies in the brain at a point in time 4 hours or 6 hours after ingestion of an agent for increasing the concentration of total ketone bodies can be maintained, for example, at 70% or more (preferably 80% or more) of a value of the concentration of total ketone bodies in the brain at a point in time 2 hours after ingestion of the agent for increasing the concentration of total ketone bodies.

Ingestion of the agent for increasing the concentration of total ketone bodies not only can increase the concentration of total ketone bodies in the brain but also can maintain that increased concentration for a prolonged period of time. The present invention, which can effectively supply energy to the brain, can provide promising treatment or prevention of a disease caused by insufficient energy in the brain and the like.

Diseases to which the present invention can provide promising treatment or prevention include dementia and age-associated cognitive decline (AACD). Other diseases for the treatment or prevention of which the herein-disclosed agent and/or oil and/or fat can be used include mild cognitive impairment (MCI), age associated memory impairment (AAMI), brain cancer, epilepsy, glucose transporter 1 deficiency syndrome (GLUT-1DS), autism, depression, anxiety disorder, and the like.

The term "dementia" as used in the present invention means a state where the cognitive function once normally obtained continually declines down to a level of interfering with daily life and social life due to an acquired brain disorder. There is no particular limitation for the type of dementia in the present invention, but examples thereof include Alzheimer's dementia, cerebrovascular-type dementia, Lewy body dementia, frontotemporal dementia, and the like. Among these, Alzheimer's dementia can be a particularly promising target of the present invention. There is no particular limitation for a causative disease for dementia which can be targeted in the present invention, but examples thereof include cerebrovascular disorders (cerebral infarction and the like), Parkinson's disease, Huntington's disease, and the like.

The term "mild cognitive impairment (MCI)" as used herein is as defined in the 2003 MCI key symposium, and refers to a state where a patient and third parties (family and the like) have reported cognitive decline, and her/his cognitive function is not normal, but does not meet the diagnostic criteria of dementia. That is, the term means a state where a patient is capable of maintaining basic daily life, and her/his impairment in complicated daily life functions remains mild.

The term "age-associated memory impairment (AAMI)" as used herein is a memory disorder in healthy elderly adults, and refers to a state where a patient aged 50 years or more who reports a memory disorder in daily life has a memory-test result lower by one standard deviation (SD) or more than the mean value of young healthy persons, but does not have dementia, as defined in 1986 by Crook et al. at the National Institute of Mental Health.

The term "age-associated cognitive decline (AACD)" as used in the present invention represents a concept proposed by Levy at the International Psychogeriatric Association in 1994. Unlike AAMI, the term means that "multiple cognitive functions such as attentional, reasoning, linguistic, and visuospatial cognition in addition to memory and learning have declined by one standard deviation (SD) or more as compared with the normal average of cognitive function tests in which age and education are taken into account. This concept can encompass both healthy aged states and pre-dementia states.

The term "cognitive decline" as used in the present invention refers to a state where cognitive functions once normally obtained continuously decline due to an acquired brain disorder, but no interference is caused with daily life and social life. There is no particular limitation for the type of mild cognitive impairment, but examples thereof include mild cognitive impairment (MCI), age-associated memory impairment (AAMI), age-associated cognitive decline (AACD), and the like. Among these, age-associated memory impairment (AAMI) can be a particularly promising target.

Cancer cells generally consume a large amount of glucose. Taking advantage of this, ingesting the agent for increasing the concentration of total ketone bodies while minimizing the amount of glucose available for the cancer cells in the brain can supply energy to normal cells while preventing growth of cancer cells. Therefore, the herein-disclosed agent and/or oil and/or fat can provide promising treatment or prevention of brain cancer while preventing the onset of cerebral dysfunction in a patient.

The term "treatment" as used in the present invention means delaying progression of, dementia or age-associated cognitive decline, as well as mitigating, alleviating, ameliorating, and healing symptoms. The term "prevention" means suppressing or delaying the onset of dementia or age-associated cognitive decline.

The concentration of total ketone bodies in the brain can be maintained over a prolonged period of time by ingesting the agent for increasing the concentration of total ketone bodies. Therefore, a decrease in the concentration of total ketone bodies in the brain can be prevented even when an energy source is not ingested between meals. Therefore, the concentration of total ketone bodies in the brain can be appropriately maintained when the agent for increasing the concentration of total ketone bodies is ingested at an appropriate timing along with a usual meal. For example, ingesting the agent for increasing the concentration of total ketone bodies 2 to 4 times per day can maintain the concentration of total ketone bodies in the brain for one day.

The agent for increasing the concentration of total ketone bodies may be applied to human and non-human mammals (canine, feline, livestock (bovine, swine, sheep, goat, and the like), equine, panda, and the like).

The mode of administration of the agent for increasing the concentration of total ketone bodies can be selected depending on the form of formulation, the age and sex of a subject to be dosed, the severity of conditions of a subject to be dosed, and other conditions.

There is no particular limitation for a method of manufacturing the agent for increasing the concentration of total ketone bodies, but a method involving mixing, stirring, and the like of the aforementioned components in an appropriate fashion; and any known formulation methods can be used.

### <Oil and/or fat composition>

The oil and/or fat composition comprises an oil and/or fat which includes the 3 types of MCFAs for use in the present invention as constituent fatty acids thereof. Specifically, an oil and/or fat contained in the oil and/or fat composition includes 50 mass% or more to 75 mass% or less of an MCFA having a carbon number of 8, 0 mass% or more to less than 25 mass% of an MCFA having a carbon number of 10, and 25 mass% or more to 50 mass% or less of an MCFA having a carbon number of 12 with respect to the total amount of MCFAs contained in the oil and/or fat composition, as constituent fatty acids thereof. Preferably, an oil and/or fat contained in the oil and/or fat composition consists of 50 mass% or more to 75 mass% or less of an MCFA having a carbon number of 8, 0 mass% or more to less than 25 mass% of an MCFA having a carbon number of 10, and 25 mass% or more to 50 mass% or less of an MCFA having a carbon number of 12 with respect to the total amount of MCFAs contained in the oil and/or fat composition, as constituent fatty acids thereof (i.e., no fatty acid is included other than the 3 types of MCFAs for use in the present invention in the aforementioned amounts as constituent fatty acids of an oil and/or fat contained in the oil and/or fat composition).

The oil and/or fat composition can be ingested in place of the agent for increasing the concentration of total ketone bodies or along with the agent for increasing the concentration of total ketone bodies to maintain a high concentration of total ketone bodies in the brain.

In view of more easily maintaining the concentration of total ketone bodies for a prolonged period of time, the oil and/or fat composition includes 50 mass% or more, preferably 55 mass% or more, even more preferably 58 mass% or more of an MCFA having a carbon number of 8 with respect to the total amount of MCFAs contained in the oil and/or fat composition. In view of sufficiently blending MCFAs other than an MCFA having a carbon number of 8, the oil and/or fat composition includes 75 mass% or less, more preferably 69 mass% or less, and even more preferably 66 mass% or less of an MCFA having a carbon number of 8 with respect to the total amount of MCFAs contained in the oil and/or fat composition.

In view of more easily maintaining the concentration of total ketone bodies for a prolonged period of time, the oil and/or fat composition includes 0 mass% or more of an MCFA having a carbon number of 10 with respect to the total amount of MCFAs contained in the oil and/or fat composition. The oil and/or fat composition does not need to include an MCFA having a carbon number of 10. In view of sufficiently blending MCFAs other than an MCFA having a carbon number of 10, the oil and/or fat composition includes less than 25 mass%, 10 mass% or less, more preferably 7.0 mass% or less, and even more preferably 5.0 mass% or less of an MCFA having a carbon number of 10 with respect to the total amount of MCFAs contained in the oil and/or fat composition. In view of more readily achieving the effects of the present invention, the oil and/or fat composition does not need to include an MCFA having a carbon number of 10.

In view of more easily maintaining the concentration of total ketone bodies for a prolonged period of time, the oil and/or fat composition includes 25 mass% or more, preferably 31 mass% or more, and even more preferably 34 mass% or more of an MCFA having a carbon number of 12 with respect to the total amount of MCFAs contained in the oil and/or fat composition. In view of sufficiently blending MCFAs other than an MCFA having a carbon number of 12, the oil and/or fat composition includes 50 mass% or less, more preferably 45 mass% or less, and even more preferably 42 mass% or less of an MCFA having a carbon number of 12 with respect to the total amount of MCFAs contained in the oil and/or fat composition.

An oil and/or fat contained the oil and/or fat composition includes a triglyceride including any one of more of the 3 types of MCFAs for use in the present invention and a long-chain fatty acid as constituent fatty acids thereof (for example, a linear long-chain fatty acid having a carbon number of 14 to 22), i.e., an MLCT; and/or a triglyceride in which constituent fatty acids thereof are all MCFAs, i.e., an MCT. As an oil and/or fat contained in the oil and/or fat composition, an MCT is particularly preferred in which constituent fatty acids thereof are each any one of the 3 types of MCFAs for use in the present invention.

An oil and/or fat contained in the oil and/or fat composition preferably includes an MLCT and/or an MCT, and preferably includes 30 mass% or more of an MCT, more preferably includes 60 mass% or more of an MCT, even more preferably includes 80 mass% or more of an MCT, and most preferably includes an MCT only with respect to the total amount of MLCT and MCT. The oil and/or fat composition in particular preferably consists of an MCT, and most preferably consists of an MCT including only the 3 types of MCFAs for use in the present invention.

Fatty acids of an oil and/or fat contained in the oil and/or fat composition may be of the same type, or may be of different types. Further, MLCTs and MCTs can be manufactured by a method similar to the aforementioned method of manufacturing acylglycerol.

The oil and/or fat composition may include any component unless the effects of the 3 types of MCFAs for use in the present invention are not impaired. Examples of such a component include proteins, lipids, available carbohydrates, vitamins, minerals, sweetening agents, anti-oxidative agents, emulsifying agents, various seasonings, swelling agents, flavoring agents, coloring agents, and the like. The types and blending amounts of these components can be appropriately selected depending on the desired effect.

The contents of the 3 types of MCFAs for use in the present invention contained in the oil and/or fat composition can be determined by gas chromatography.

### <Pharmaceutical composition>

The herein described agent for increasing the concentration of total ketone bodies and the herein described oil and/or fat composition can be applied to the manufacturing of a pharmaceutical composition for use in treatment or prevention of dementia or age-associated cognitive decline. A pharmaceutical composition for use in treatment or prevention of dementia or age-associated cognitive decline including the herein described agent for increasing the concentration of total ketone bodies and/or the herein described oil and/or fat composition (hereinafter may also be referred to as a "pharmaceutical composition for use according to an embodiment of the present invention") has less risk of an adverse effect, and can preferably be used as a pharmaceutical product suitable for long-term administration.

The pharmaceutical composition for use according to an embodiment of the present invention may be in any form of solid, liquid, or gel.

There is no particular limitation for the form of the pharmaceutical composition for use according to an embodiment of the present invention, and it may be prepared as either an oral pharmaceutical composition or a parenteral pharmaceutical composition. In view of easy long-term ingestion, the pharmaceutical composition for use according to an embodiment of the present invention is preferably an oral pharmaceutical composition.

Forms of an oral pharmaceutical composition include, for example, formulations such as capsules, tablets, pills, powders, fine granules, granules, solutions, and syrups. Forms of a parenteral pharmaceutical composition include, for example, formulations such injectables and infusions.

The pharmaceutical composition for use according to an embodiment of the present invention preferably includes the herein described agent for increasing the concentration of total ketone bodies and/or the herein described oil and/or fat composition as well as a pharmacologically and pharmaceutically acceptable additive. For a "pharmacologically and pharmaceutically acceptable additive," a material can be used which is commonly used as an excipient and the like in the field of formulation, and which does not react with an active ingredient (i.e., an MCFA) included in the agent for increasing the concentration of total ketone bodies and the oil and/or fat composition.

The dosage amount of the pharmaceutical composition for use according to an embodiment of the present invention may be appropriately selected depending on the purpose of administration (prevention or treatment), the method of administration, the period of administration, and other various conditions (for example, the condition, age, and weight of a patient).

For oral administration, the lower limit of the dosage amount of the pharmaceutical composition for use according to an embodiment of the present invention can be preferably set at 0.02 g/Kg of weight/day or more, more preferably 0.08 g/Kg of weight/day or more in terms of the amount of the 3 types of MCFAs for use in the present invention. The upper limit can be preferably set at 0.70 g/Kg of weight/day or less, more preferably 0.45 g/Kg of weight/day or less in terms of the amount of the 3 types of MCFAs for use in the present invention.

For parenteral administration, the lower limit of the dosage amount of the pharmaceutical composition for use according to an embodiment of the present invention can be preferably set at 0.026 g/Kg of weight/day or more, more preferably 0.09 g/Kg of weight/day or more in terms of the amount of the 3 types of MCFAs for use in the present invention. The upper limit can be preferably set at 0.072 g/Kg of weight/day or less, more preferably 0.56 g/Kg of weight/day or less in terms of the amount of the 3 types of MCFAs for use in the present invention. It is noted that the above dosage amounts are preferably administered over a period of several hours (for example, over a period of 4 to 8 hours) in the case of parenteral administration.

The pharmaceutical composition for use according to an embodiment of the present invention, which has less risk of an adverse effect and less likely interacts with a common active ingredient, may be used in combination with an existing drug (a therapeutic drug of Alzheimer's disease, a cholinesterase inhibitor, an N-methyl-D-asparagine receptor antagonist, and the like). In a case where an existing drug used in combination with the pharmaceutical composition for use according to an embodiment of the present invention is a therapeutic drug of Alzheimer's disease, the dose of the existing drug can likely be decreased. This may reduce an adverse effect of the existing drug.

The pharmaceutical composition for use according to an embodiment of the present invention has less risk of an adverse effect, and is thus suitable for long-term administration. There is no particular limitation for a period of administration, but long-term administration is preferred when amelioration in symptoms is observed upon prevention and treatment of dementia or age-associated cognitive decline. Administration may be performed at every several hours during the above period of time, or may be performed with an interval (for example, one day to several days).

### <Food product composition>

The herein described agent for increasing the concentration of total ketone bodies and the herein described oil and/or fat composition can be applied to the manufacturing of a food product composition for use in treatment or prevention of dementia or age-associated cognitive decline. MCFAs as active ingredients of the agent for increasing the concentration of total ketone bodies and the oil and/or fat composition not only have less risk of an adverse effect but are also less likely to spoil the flavor or taste of a food product. Therefore, a food product composition for use in treatment or prevention of dementia or age-associated cognitive decline including the agent for increasing the concentration of total ketone bodies and/or the oil and/or fat composition (hereinafter may also be referred to as the "food product composition for use according to an embodiment of the present invention") can be preferably used as an easily ingestible food product.

The food product composition for use according to an embodiment of the present invention may be in any form of solid, liquid, or gel.

Forms of the food product composition for use according to an embodiment of the present invention include supplements, common food products, animal food products, and animal feedstuffs.

There is no particular limitation for the form of a supplement, and the form may be either a solid formulation or a liquid formulation. Examples include formulations such as tablets, coated tablets, capsules, granules, powders, powered formulations, sustained release formulations, suspensions, emulsions, oral liquid formulations, sugar-coated tablets, pills, fine granules, syrups, and elixirs.

There is no particular limitation for the form of a common food product, but examples thereof include, for example, pastry/confectionary (bread, cake, cookie, biscuit, doughnut, muffin, scone, chocolate, snack, whipped cream, ice cream, and the like), drinks (fruit juice drink, nutritional drink, isotonic drink, and the like), soups, seasoned and processed food products (dressing, sauce, mayonnaise, butter, margarine, processed margarine, and the like), fat spreads, shortenings, bakery mixes, stir-fry oils, frying oils, fried food products, processed meat products, frozen food products, fried food products, noodles, retort food products, liquid foods, dysphagia foods, and the like.

When the agent for increasing the concentration of total ketone bodies and/or the oil and/or fat composition are used for the manufacturing of a common food product, MCFAs present in the form of an MLCT and/or an MCT (more preferably an MCT) may be added to raw materials, or an MLCT and/or an MCT (more preferably an MCT) may be preferably used in place of an oil and/or fat in raw materials.

The lower limit of the blending amount of the 3 types of MCFAs for use in the present invention (MCFAs themselves, a fatty acid precursor of MCFAs (an MLCT, an MCT, and the like), or a mixture thereof) contained in the food product composition for use according to an embodiment of the present invention is preferably 1 mass%, and more preferably 5 mass%. The upper limit is preferably 90 mass% or less, and more preferably 60 mass% or less. Within the above ranges, the food product composition for use according to an embodiment of the present invention can be ingested comfortably like a common food product.

The food product composition for use according to an embodiment of the present invention may include a component which can be used in a food product. Examples of such a component include emulsifying agents, gelatinizing agents (glucomannan, carrageenan, and the like), saccharides, citric acid, sweetening agents, flavoring agents, and the like.

The ingestion amount of the food product composition for use according to an embodiment of the present invention can be appropriately selected depending on the purpose of ingestion (prevention or treatment), the period of ingestion, and other various conditions (for example, the condition, age, and weight of an ingesting person).

The lower limit of the ingestion amount of the food product composition for use according to an embodiment of the present invention may be set at preferably 0.008 g/Kg of weight/day or more, more preferably 0.08 g/Kg of weight/day or more in terms of the amount of the 3 types of MCFAs for use in the present invention. The upper limit may be set at preferably 3.0 g/Kg of weight/day or less, more preferably 1.5 g/Kg of weight/day or less in terms of the amount of the 3 types of MCFAs for use in the present invention.

### EXAMPLES

Below, the present invention will be described more specifically with reference to Examples, but the present invention shall not be limited to these Examples.

### <Preparation of oil and/or fat>

Oil and/or fats used in Example and Comparative Examples were prepared as follows.

### (Example 1)

An MCT (from Nisshin Oillio Group, Ltd.) was provided as an oil and/or fat for use in Example 1, in which a triglyceride includes constituent fatty acids consisting of octanoic acid (corresponding to an MCFA having a carbon number of 8) and lauric acid (corresponding to an MCFA having a carbon number of 12) with a mass ratio of octanoic acid:lauric acid = 65:35. The above oil and/or fat corresponds to the agent for increasing the concentration of total ketone bodies or the oil and/or fat composition present in the pharmaceutical composition for use and in the food product for use according to the present invention.

### (Comparative Example 1)

A refined coconut oil (Product name "Nisshin Coconut Oil", from Nisshin Oillio Group, Ltd.) was provided as an oil and/or fat for use in Comparative Example 1. In the above oil and/or fat, a triglyceride has constituent fatty acids consisting of octanoic acid, decanoic acid (corresponding to an MCFA having a carbon number of 10), lauric acid, and other fatty acids with a mass ratio of octanoic acid:decanoic acid:lauric acid:other fatty acids = 8.3:6.4:46.7:38.6. It is noted that in the above oil and/or fat, the mass ratio of octanoic acid, decanoic acid, and lauric acid is octanoic acid:decanoic acid:lauric acid = 14:10:76 when the total mass of octanoic acid, decanoic acid, and lauric acid is taken as 100.

### (Comparative Example 2)

An MCT (from Nisshin Oillio Group, Ltd.) was provided as an oil and/or fat for use in Comparative Example 2, in which a triglyceride includes constituent fatty acids consisting only of octanoic acid.

### (Comparative Example 3)

An MCT (from Nisshin Oillio Group, Ltd.) was provided as an oil and/or fat for use in Comparative Example 3, in which a triglyceride includes constituent fatty acids consisting of octanoic acid (corresponds to an MCFA having a carbon number of 8), decanoic acid (corresponding to an MCFA having a carbon number of 10), and lauric acid (corresponding to an MCFA having a carbon number of 12) with a mass ratio of octanoic acid:decanoic acid:lauric acid = 40:25:35.

Table 1 shows the ratio (unit: mass%) of octanoic acid, decanoic acid, and lauric acid in constituent fatty acids for each of these oil and/or fats when the total mass of octanoic acid, decanoic acid, and lauric acid is taken as 100.

**[Table 1]**

| Table 1 | | | |
|---|---|---|---|
| | Octanoic acid (C8 MCFA) | Decanoic acid (C10 MCFA) | Lauric acid (C12 MCFA) |
| Example 1 | 65 | 0 | 35 |
| Comparative Example 1 | 14 | 10 | 76 |
| Comparative Example 2 | 100 | 0 | 0 |
| Comparative Example 3 | 40 | 25 | 35 |

### <Administration tests of oil and/or fat to rats (1)>

In the tests, used are 7-week old Sprague-Dawley male rats (CLEA Japan, Inc.). Rats were individually caged, and kept in a rearing room at a room temperature of 23 ± 1°C and a relative humidity of 50 ± 5% under a light-dark cycle of 12 hours. Rats were given ad libitum access to water and food (Product name "CE-2" from CLEA Japan, Inc.).

Rats were grouped into three groups as described below so that the mean weights of rats were uniform for each of these groups before the start of the tests. It is noted that 6 rats from each of the groups were used.
(1) A group in which the oil and/or fat from Example 1 is given
(2) A group in which the oil and/or fat from Comparative Example 1 is given
(3) A group in which the oil and/or fat from Comparative Example 2 is given

The amount of feed ingestion during the dark period was weighed for each individual. The mean weight of feed ingestion over 2 days was computed, and two-thirds of the mean weight of feed ingestion during the dark period was given one day before administration of an oil and/or fat for each test group. On the day of administering an oil and/or fat for each test group, a rat which had fasted for 2 hours was given the oil and/or fat for each test group (10 mg/g weight) via an oral administration probe when the cycle was at the light period, and then forced to abstain from water.

At each of the time points of before administration of an oil and/or fat and 2 hours, 4 hours, and 6 hours after administration, a rat was irradiated with a microwave at the head for 1.5 seconds using a microwave applicator (Product name "MMW-5" from Muromachi Kikai Co., Ltd.) to inactivate various enzymes, and then the hippocampus was excised, and immediately cryopreserved at -80°C. All samples were sonicated and homogenized in a β-HB assay buffer (BioVision Inc.) solution.

### <Administration tests of oil and/or fat to rats (2)>

In the tests, used were 7-week old Sprague-Dawley male rats (CLEA Japan, Inc.). Rats were individually caged, and kept in a rearing room at a room temperature of 23 ± 1°C and a relative humidity of 50 ± 5% of under a light-dark cycle of 12 hours. Rats were given ad libitum access to water and food (Product name "CE-2" from CLEA Japan, Inc.).

Rats were grouped into 4 groups as described below so that the mean weights of rats were uniform for each of these groups before the start of the tests. It is noted that 6 rats were used in each of the groups.
(1) A group in which the oil and/or fat from Example 1 is given
(2) A group in which the oil and/or fat from Comparative Example 1 is given
(3) A group in which the oil and/or fat from Comparative Example 2 is given
(4) A group in which the oil and/or fat from Comparative Example 3 is given

The amount of feed ingestion during the dark period was weighed for each individual. The mean weight of feed ingestion over 2 days was computed, and two-thirds of the mean weight of feed ingestion during the dark period was given one day before administration of an oil and/or fat for each test group. On the day of administering an oil and/or fat for each test group, a rat which had fasted for 2 hours was given the oil and/or fat for each test group (2.5 mg/g weight) via an oral administration probe when the cycle was at the light period, and then forced to abstain from water.

At each of the time points of before administration of an oil and/or fat and 2 hours and 4 hours after administration, a rat was irradiated with a microwave at the head for 1.5 seconds using a microwave applicator (Product name "MMW-5" from Muromachi Kikai Co., Ltd.) to inactivate various enzymes, and then the hippocampus was excised, and immediately cryopreserved at -80°C. All samples were sonicated and homogenized in a β-HB assay buffer (BioVision Inc.) solution.

### <Measurement of concentration of total ketone bodies in hippocampus>

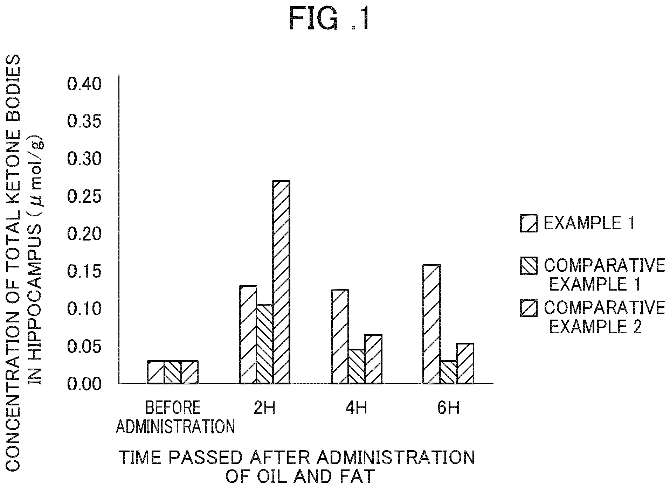

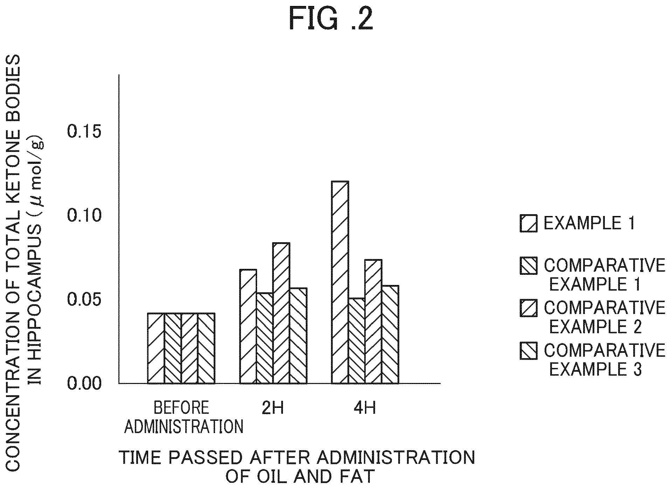

The concentration of total ketone bodies (i.e., the total concentration of acetoacetic acid and 3-hydroxybutyric acid) in a hippocampus sample obtained above was measured using a kit (Product name "Auto Wako total ketone bodies," from Wako Pure Chemical Industries, Ltd.). Fig. 1 shows results from "<Administration tests of oil and/or fat to rats (1)>," and Fig. 2 shows results from "<Administration tests of oil and/or fat to rats (2)>." It is noted that the concentrations of total ketone bodies shown in the figures each represent the mean value from each group.

As understood from Fig. 1, the concentration of total ketone bodies in the hippocampus was increased after administration, and further the concentration of total ketone bodies was then maintained for 6 hours after administration for rats administered with the oil and/or fat from Example 1. This demonstrates that the agent for increasing the concentration of total ketone bodies according to an embodiment of the present invention or the oil and/or fat composition according to an embodiment of the present invention not only can increase the concentration of total ketone bodies in the brain but also can maintain that concentration for 6 hours or longer.

In contrast, as understood from Fig. 1, the concentrations of total ketone bodies in the hippocampus were increased after administration, but that concentration significantly decreased over time after administration for rats administered with the oil and/or fats from Comparative Examples 1 and 2. Further, the concentrations of total ketone bodies at a point in time of 6 hours after administration were at the substantially same level as that before administration.

As understood from Fig. 2, the concentration of total ketone bodies in the hippocampus was increased after administration, and further the concentration of total ketone bodies was then maintained for at least 4 hours after administration for rats administered with the oil and/or fat from Example 1.

It is noted that Comparative Example 3 showed a similar trend as Example 1 when the percentage of decanoic acid in the constituent fatty acids of the triglyceride was changed to less than 25 mass%, and the percentages of octanoic acid and lauric acid were increased accordingly, although this is not shown in Fig. 2.

### <Method of manufacturing gelatinous composition>

The following method was performed using raw materials described below to prepare a food product composition for use according to an embodiment of the present invention as a gelatinous composition. First, raw materials other than an oil and/or fat were dispersed in water, and then heated to a dissolution temperature (90°C) of a gelatinizing agent, and the raw materials were then mixed and dissolved to obtain a uniform solution. Subsequently, an oil and/or fat was charged into the above solution, and emulsified with a mixer (instead, a homogenizer and the like may be used) to obtain an oil-in-water emulsion. Then, 15 g of the resulting oil-in-water emulsion (1400 g) was dispensed and sealed into an aluminum pouch bag while maintaining it at or above the solidifying temperature of the gelatinizing agent, and then cooled to allow gelation to obtain a gelatinous composition. It is noted that the gelatinous composition was able to be sterilized by any of the following methods: a method involving filling a container with the gelatinous composition, sealing the container, and then performing heat sterilization; a method involving filling a container with the gelatinous composition while performing heat sterilization; and a method involving performing heat sterilization before filling, and then performing aseptic filling.

### (Raw materials of gelatinous composition)

Oil and/or fat (40 mass%): an MCT (from Nisshin Oillio Group, Ltd.) was provided as an oil and/or fat, in which a triglyceride includes constituent fatty acids consisting of octanoic acid (corresponds to an MCFA having a carbon number of 8) and lauric acid (corresponding to an MCFA having a carbon number of 12) with a mass ratio of octanoic acid:lauric acid = 65:35. The above oil and/or fat corresponds to the agent for increasing the concentration of total ketone bodies or the oil and/or fat composition present in the pharmaceutical composition for use and in the food product for use according to the present invention.
Emulsifying agent (1.5 mass%): glycerin fatty acid ester, Product name "POEM J-0081HV," from Riken Vitamin Co., Ltd. Gelatinizing agent (1) (0.3 mass%): carrageenan, Product name "GENUGEL WG-108," from Sansho Co., Ltd.
Gelatinizing agent (2) (0.3 mass%): xanthan gum, Product name "ECHO GUM" from DSP Gokyo Food & Chemical Co., Ltd. Gelatinizing agent (3) (0.3 mass%): agar, Product name "Ina Agar S-6" from Ina Food Industry Co., Ltd.
Gelatinizing agent (4) (0.5 mass%): Locust bean gum, Product name "Meyprodyn 200," from Sansho Co., Ltd.
Citric acid (0.2 mass%): Product name "citric acid (anhydrous)," from Fuso Chemical Co., Ltd.
Yogurt flavor (0.2 mass%): Product name "HL01043," from Ogawa & Co., Ltd.
Sweetening agent (0.2 mass%): Product name "Preziron SU-10" from Tsuruya Chemical Industries, Ltd.
Water (56.5 mass%)

The gelatinous composition obtained above includes 6 g of an oil and/or fat (corresponding to the agent for increasing the concentration of total ketone bodies or the oil and/or fat compositionpresent in the pharmaceutical composition for use and in the food product for use according to the present invention) per bag (15 g). For a human having a weight of 60 Kg, ingestion of the agent for increasing the concentration of total ketone bodies or the oil and/or fat composition can be achieved conveniently by ingesting the aforementioned gelatinous composition as a food product 2 to 4 times per day to provide the effects of the present invention.

## Claims

1. A pharmaceutical composition for use in treatment or prevention of dementia or age-associated cognitive decline, the pharmaceutical composition comprising
a) an agent for use in a method of increasing the concentration of total ketone bodies, the agent comprising 40 mass% or more to 75 mass% or less of a medium-chain fatty acid having a carbon atom number of 8, 0 mass% or more to less than 25 mass% of a medium-chain fatty acid having a carbon number of 10, and 25 mass% or more to 60 mass% or less of a medium-chain fatty acid having a carbon number of 12 with respect to the total amount of medium-chain fatty acids; and/or
b) an oil and/or fat composition comprising an oil and/or fat having constituent fatty acids including 50 mass% or more to 75 mass% or less of a medium-chain fatty acid having a carbon number of 8, 0 mass% or more to less than 25 mass% of a medium-chain fatty acid having a carbon number of 10, and 25 mass% or more to 50 mass% or less of a medium-chain fatty acid having a carbon number of 12 with respect to the total amount of medium-chain fatty acids, wherein the medium-chain fatty acids are present in the form of acylglycerols.

2. A pharmaceutical composition for use according to claim 1, wherein the agent for use in a method of increasing the concentration of total ketone bodies comprises 60 mass% or more to 70 mass% or less of a medium-chain fatty acid having a carbon number of 8, and 30 mass% or more to 40 mass% or less of a medium-chain fatty acid having a carbon number of 12 with respect to the total amount of medium-chain fatty acids.

3. A pharmaceutical composition for use according to claim 1 or 2, wherein the agent increases the concentration of total ketones in a brain.

4. A pharmaceutical composition for use according to claim 1 to 3, wherein the agent for use in a method of increasing the concentration of total ketone bodies comprises a medium-chain fatty-acid triglyceride as a source of medium-chain fatty acids.

5. A pharmaceutical composition for use according to claim 1, wherein the oil and/or fat composition comprises an oil and/or fat having constituent fatty acids consisting of 50 mass% or more to 75 mass% or less of a medium-chain fatty acid having a carbon number of 8, 0 mass% or more to 10 mass% or less of a medium-chain fatty acid having a carbon number of 10, and 25 mass% or more to 50 mass% or less of a medium-chain fatty acid having a carbon number of 12 with respect to the total amount of medium-chain fatty acids.

6. A food product composition for use in treatment or prevention of dementia or age-associated cognitive decline, the food product composition comprising
a) an agent for use in a method of increasing the concentration of total ketone bodies, the agent comprising 40 mass% or more to 75 mass% or less of a medium-chain fatty acid having a carbon atom number of 8, 0 mass% or more to less than 25 mass% of a medium-chain fatty acid having a carbon number of 10, and 25 mass% or more to 60 mass% or less of a medium-chain fatty acid having a carbon number of 12 with respect to the total amount of medium-chain fatty acids; and/or;
b) oil and/or fat composition comprising an oil and/or fat having constituent fatty acids including 50 mass% or more to 75 mass% or less of a medium-chain fatty acid having a carbon number of 8, 0 mass% or more to less than 25 mass% of a medium-chain fatty acid having a carbon number of 10, and 25 mass% or more to 50 mass% or less of a medium-chain fatty acid having a carbon number of 12 with respect to the total amount of medium-chain fatty acids, wherein the medium-chain fatty acids are present in the form of acylglycerols.

7. A food product composition for use according to claim 6, wherein the agent for use in a method of increasing the concentration of total ketone bodies comprises 60 mass% or more to 70 mass% or less of a medium-chain fatty acid having a carbon number of 8, and 30 mass% or more to 40 mass% or less of a medium-chain fatty acid having a carbon number of 12 with respect to the total amount of medium-chain fatty acids.

8. A food product composition for use according to claim 6 or 7, wherein the agent for use in a method of increasing the concentration of total ketone bodies increases the concentration of total ketones in a brain.

9. A food product composition for use according to any one of claims 6 to 8, wherein the agent for use in a method of increasing the concentration of total ketone bodies comprises a medium-chain fatty-acid triglyceride as a source of medium-chain fatty acids.

10. A food product composition for use according to claim 6, wherein the oil and/or fat composition comprises an oil and/or fat having constituent fatty acids consisting of 50 mass% or more to 75 mass% or less of a medium-chain fatty acid having a carbon number of 8, 0 mass% or more to 10 mass% or less of a medium-chain fatty acid having a carbon number of 10, and 25 mass% or more to 50 mass% or less of a medium-chain fatty acid having a carbon number of 12 with respect to the total amount of medium-chain fatty acids.

## Patentansprüche

1. Pharmazeutische Zusammensetzung zur Verwendung bei der Behandlung oder Vorbeugung von Demenz oder altersbedingtem kognitiven Verfall, wobei die pharmazeutische Zusammensetzung umfasst
a) ein Mittel zur Verwendung in einem Verfahren zur Erhöhung der Konzentration der gesamten Ketonkörper, wobei das Mittel 40 Massen-% oder mehr bis 75 Massen-% oder weniger einer mittelkettigen Fettsäure mit einer Kohlenstoffatomzahl von 8, 0 Massen-% oder mehr bis weniger als 25 Massen-% einer mittelkettigen Fettsäure mit einer Kohlenstoffzahl von 10, und 25 Massen-% oder mehr bis 60 Massen-% oder weniger einer mittelkettigen Fettsäure mit einer Kohlenstoffzahl von 12 umfasst, bezogen auf die Gesamtmenge der mittelkettigen Fettsäuren; und/oder
b) eine Öl- und/oder Fettzusammensetzung, umfassend ein Öl und/oder Fett mit Fettsäuren als Bestandteilen, die 50 Massen-% oder mehr bis 75 Massen-% oder weniger einer mittelkettigen Fettsäure mit einer Kohlenstoffzahl von 8, 0 Massen-% oder mehr bis weniger als 25 Massen-% einer mittelkettigen Fettsäure mit einer Kohlenstoffzahl von 10, und 25 Massen-% oder mehr bis 50 Massen-% oder weniger einer mittelkettigen Fettsäure mit einer Kohlenstoffzahl von 12 beinhalten, bezogen auf die Gesamtmenge der mittelkettigen Fettsäuren, wobei die mittelkettigen Fettsäuren in Form von Acyl-Glycerinen vorliegen.

2. Pharmazeutische Zusammensetzung zur Verwendung gemäß Anspruch 1, wobei das Mittel zur Verwendung in einem Verfahren zur Erhöhung der Konzentration der gesamten Ketonkörper 60 Massen-% oder mehr bis 70 Massen-% oder weniger einer mittelkettigen Fettsäure mit einer Kohlenstoffzahl von 8 und 30 Massen-% oder mehr bis 40 Massen-% oder weniger einer mittelkettigen Fettsäure mit einer Kohlenstoffzahl von 12 bezogen auf die Gesamtmenge an mittelkettigen Fettsäuren umfasst.

3. Pharmazeutische Zusammensetzung zur Verwendung gemäß Anspruch 1 oder 2, wobei das Mittel die Konzentration von Gesamtketonen in einem Gehirn erhöht.

4. Pharmazeutische Zusammensetzung zur Verwendung nach einem der Ansprüche 1 bis 3, wobei das Mittel zur Verwendung in einem Verfahren zur Erhöhung der Konzentration der gesamten Ketonkörper ein mittelkettiges Fettsäure-Triglycerid als eine Quelle für mittelkettige Fettsäuren umfasst.

5. Pharmazeutische Zusammensetzung zur Verwendung gemäß Anspruch 1, wobei die Öl- und/oder Fettzusammensetzung ein Öl und/oder Fett mit Fettsäuren als Bestandteile umfasst, die aus 50 Massen-% oder mehr bis 75 Massen-% oder weniger einer mittelkettigen Fettsäure mit einer Kohlenstoffzahl von 8, 0 Massen-% oder mehr bis 10 Massen-% oder weniger einer mittelkettigen Fettsäure mit einer Kohlenstoffzahl von 10, und 25 Massen-% oder mehr bis 50 Massen-% oder weniger einer mittelkettigen Fettsäure mit einer Kohlenstoffzahl von 12 bestehen, bezogen auf die Gesamtmenge der mittelkettigen Fettsäuren.

6. Nahrungsmittelprodukt-Zusammensetzung zur Verwendung bei der Behandlung oder Vorbeugung von Demenz oder altersbedingtem kognitivem Verfall, wobei das Nahrungsmittelprodukt umfasst
a) ein Mittel zur Verwendung in einem Verfahren zur Erhöhung der Konzentration der gesamten Ketonkörper, wobei das Mittel 40 Massen-% oder mehr bis 75 Massen-% oder weniger einer mittelkettigen Fettsäure mit einer Kohlenstoffatomzahl von 8, 0 Massen-% oder mehr bis weniger als 25 Massen-% einer mittelkettigen Fettsäure mit einer Kohlenstoffzahl von 10, und 25 Massen-% oder mehr bis 60 Massen-% oder weniger einer mittelkettigen Fettsäure mit einer Kohlenstoffzahl von 12 umfasst, bezogen auf die Gesamtmenge der mittelkettigen Fettsäuren; und/oder;
b) eine Öl- und/oder Fettzusammensetzung, umfassend ein Öl und/oder Fett mit Fettsäuren als Bestandteilen, die 50 Massen-% oder mehr bis 75 Massen-% oder weniger einer mittelkettigen Fettsäure mit einer Kohlenstoffzahl von 8, 0 Massen-% oder mehr bis weniger als 25 Massen-% einer mittelkettigen Fettsäure mit einer Kohlenstoffzahl von 10, und 25 Massen-% oder mehr bis 50 Massen-% oder weniger einer mittelkettigen Fettsäure mit einer Kohlenstoffzahl von 12 beinhalten, bezogen auf die Gesamtmenge der mittelkettigen Fettsäuren, wobei die mittelkettigen Fettsäuren in Form von Acyl-Glycerinen vorliegen.

7. Nahrungsmittelprodukt-Zusammensetzung zur Verwendung nach Anspruch 6, wobei das Mittel zur Verwendung in einem Verfahren zur Erhöhung der Konzentration der gesamten Ketonkörper 60 Massen-% oder mehr bis 70 Massen-% oder weniger einer mittelkettigen Fettsäure mit einer Kohlenstoffzahl von 8, und 30 Massen-% oder mehr bis 40 Massen-% oder weniger einer mittelkettigen Fettsäure mit einer Kohlenstoffzahl von 12 umfasst, bezogen auf die Gesamtmenge der mittelkettigen Fettsäuren.

8. Nahrungsmittelprodukt-Zusammensetzung zur Verwendung nach Anspruch 6 oder 7, wobei das Mittel zur Verwendung in einem Verfahren zur Erhöhung der Konzentration der gesamten Ketonkörper die Konzentration von Gesamtketonen in einem Gehirn erhöht.

9. Nahrungsmittelprodukt-Zusammensetzung zur Verwendung nach einem der Ansprüche 6 bis 8, wobei das Mittel zur Verwendung in einem Verfahren zur Erhöhung der Konzentration der gesamten Ketonkörper ein mittelkettiges Fettsäure-Triglycerid als eine Quelle für mittelkettige Fettsäuren umfasst.

10. Nahrungsmittelprodukt-Zusammensetzung zur Verwendung gemäß Anspruch 6, wobei die Öl- und/oder Fettzusammensetzung ein Öl und/oder Fett mit Fettsäuren als Bestandteile umfasst, die aus 50 Massen-% oder mehr bis 75 Massen-% oder weniger einer mittelkettigen Fettsäure mit einer Kohlenstoffzahl von 8, 0 Massen-% oder mehr bis 10 Massen-% oder weniger einer mittelkettigen Fettsäure mit einer Kohlenstoffzahl von 10, und 25 Massen-% oder mehr bis 50 Massen-% oder weniger einer mittelkettigen Fettsäure mit einer Kohlenstoffzahl von 12 bestehen, bezogen auf die Gesamtmenge der mittelkettigen Fettsäuren.

## Revendications

1. Composition pharmaceutique destinée à être utilisée dans le traitement ou la prévention de la démence ou du déclin cognitif lié à l'âge, la composition pharmaceutique comprenant
a) un agent destiné à être utilisé dans un procédé d'augmentation de la concentration de corps cétoniques totaux, l'agent comprenant 40% en masse ou plus à 75% en masse ou moins d'un acide gras à chaîne moyenne ayant un nombre d'atomes de carbone de 8, 0% en masse ou plus à moins de 25% en masse d'un acide gras à chaîne moyenne ayant un nombre de carbones de 10, et 25% en masse ou plus à 60% en masse ou moins d'un acide gras à chaîne moyenne ayant un nombre de carbones de 12 par rapport à la quantité totale d'acides gras à chaîne moyenne ; et/ou
b) une composition d'huile et/ou de matière grasse comprenant une huile et/ou une matière grasse ayant des acides gras constitutifs comportant 50% en masse ou plus à 75% en masse ou moins d'un acide gras à chaîne moyenne ayant un nombre de carbones de 8, 0% en masse ou plus à moins de 25% en masse d'un acide gras à chaîne moyenne ayant un nombre de carbones de 10, et 25% en masse ou plus à 50% en masse ou moins d'un acide gras à chaîne moyenne ayant un nombre de carbones de 12 par rapport à la quantité totale d'acides gras à chaîne moyenne, où les acides gras à chaîne moyenne sont présents sous forme d'acylglycérols.

2. Composition pharmaceutique destinée à être utilisée selon la revendication 1, dans laquelle l'agent destiné à être utilisé dans un procédé d'augmentation de la concentration de corps cétoniques totaux comprend 60% en masse ou plus à 70% en masse ou moins d'un acide gras à chaîne moyenne ayant un nombre de carbones de 8, et 30% en masse ou plus à 40% en masse ou moins d'un acide gras à chaîne moyenne ayant un nombre de carbones de 12 par rapport à la quantité totale d'acides gras à chaîne moyenne.

3. Composition pharmaceutique destinée à être utilisée selon la revendication 1 ou 2, dans laquelle l'agent augmente la concentration de cétones totales dans un cerveau.

4. Composition pharmaceutique destinée à être utilisée selon les revendications 1 à 3, dans laquelle l'agent destiné à être utilisé dans un procédé d'augmentation de la concentration de corps cétoniques totaux comprend un triglycéride d'acide gras à chaîne moyenne comme source d'acides gras à chaîne moyenne.

5. Composition pharmaceutique destinée à être utilisée selon la revendication 1, dans laquelle la composition d'huile et/ou de matière grasse comprend une huile et/ou une matière grasse ayant des acides gras constitutifs constitués de 50% en masse ou plus à 75% en masse ou moins d'un acide gras à chaîne moyenne ayant un nombre de carbones de 8, 0% en masse ou plus à 10% en masse ou moins d'un acide gras à chaîne moyenne ayant un nombre de carbones de 10, et 25% en masse ou plus à 50% en masse ou moins d'un acide gras à chaîne moyenne ayant un nombre de carbones de 12 par rapport à la quantité totale d'acides gras à chaîne moyenne.

6. Composition de produit alimentaire destinée à être utilisée dans le traitement ou la prévention de la démence ou du déclin cognitif lié à l'âge, la composition de produit alimentaire comprenant
a) un agent destiné à être utilisé dans un procédé d'augmentation de la concentration de corps cétoniques totaux, l'agent comprenant 40% en masse ou plus à 75% en masse ou moins d'un acide gras à chaîne moyenne ayant un nombre d'atomes de carbone de 8, 0% en masse ou plus à moins de 25% en masse d'un acide gras à chaîne moyenne ayant un nombre de carbones de 10, et 25% en masse ou plus à 60% en masse ou moins d'un acide gras à chaîne moyenne ayant un nombre de carbones de 12 par rapport à la quantité totale d'acides gras à chaîne moyenne ; et/ou ;
b) une composition d'huile et/ou de matière grasse comprenant une huile et/ou une matière grasse ayant des acides gras constitutifs comportant 50% en masse ou plus à 75% en masse ou moins d'un acide gras à chaîne moyenne ayant un nombre de carbones de 8, 0% en masse ou plus à moins de 25% en masse d'un acide gras à chaîne moyenne ayant un nombre de carbones de 10, et 25% en masse ou plus à 50% en masse ou moins d'un acide gras à chaîne moyenne ayant un nombre de carbones de 12 par rapport à la quantité totale d'acides gras à chaîne moyenne, où les acides gras à chaîne moyenne sont présents sous forme d'acylglycérols.

7. Composition de produit alimentaire destinée à être utilisée selon la revendication 6, dans laquelle l'agent destiné à être utilisé dans un procédé d'augmentation de la concentration de corps cétoniques totaux comprend 60% en masse ou plus à 70% en masse ou moins d'un acide gras à chaîne moyenne ayant un nombre de carbones de 8, et 30% en masse ou plus à 40% en masse ou moins d'un acide gras à chaîne moyenne ayant un nombre de carbones de 12 par rapport à la quantité totale d'acides gras à chaîne moyenne.

8. Composition de produit alimentaire destinée à être utilisée selon la revendication 6 ou 7, dans laquelle l'agent destiné à être utilisé dans un procédé d'augmentation de la concentration de corps cétoniques totaux augmente la concentration de cétones totales dans un cerveau.

9. Composition de produit alimentaire destinée à être utilisée selon l'une quelconque des revendications 6 à 8, dans laquelle l'agent destiné à être utilisé dans un procédé d'augmentation de la concentration de corps cétoniques totaux comprend un triglycéride d'acide gras à chaîne moyenne comme source d'acides gras à chaîne moyenne.

10. Composition de produit alimentaire destinée à être utilisée selon la revendication 6, dans laquelle la composition d'huile et/ou de matière grasse comprend une huile et/ou une matière grasse ayant des acides gras constitutifs constitués de 50% en masse ou plus à 75% en masse ou moins d'un acide gras à chaîne moyenne ayant un nombre de carbones de 8, 0% en masse ou plus à 10% en masse ou moins d'un acide gras à chaîne moyenne ayant un nombre de carbones de 10, et 25% en masse ou plus à 50% en masse ou moins d'un acide gras à chaîne moyenne ayant un nombre de carbones de 12 par rapport à la quantité totale d'acides gras à chaîne moyenne.
